# EUROPEAN PATENT SPECIFICATION

(11) **EP 2 954 846 B1**
(45) Date of publication and mention of the grant of the patent: **31.01.2018**
(21) Application number: 15171422.7
(22) Date of filing: 10.06.2015
(51) Int. Cl.: A61B 8/12, A61B 8/00, A61B 8/08

(54) **SWIPE TO SEE THROUGH ULTRASOUND IMAGING FOR INTRAOPERATIVE APPLICATIONS**
WISCHEN, UM DURCH ULTRASCHALLBILDGEBUNG FÜR INTRAOPERATIVE ANWENDUNGEN HINDURCHZUSCHAUEN
BALAYAGE PERMETTANT DE VOIR GRACE A UNE IMAGERIE ULTRASONORE POUR DES APPLICATIONS INTRA-OPERATOIRES

(30) Priority: 11.06.2014 US 201462010608 P; 04.05.2015 US 201514702976
(43) Date of publication of application: 16.12.2015
(73) Proprietor: Covidien LP, Mansfield, MA 02048 (US)
(72) Inventor: TAN, Wei, 201318 Shanghai (CN)
(74) Representative: Soames, Candida Jane

(56) References cited:
- US-A1- 2010 268 086
- US-A1- 2012 157 842
- STETTEN G ET AL: "C-Mode Real-time Tomographic Reflection for a Matrix Array Ultrasound Sonic Flashlight<1>", ACADEMIC RADIOLOGY, RESTON, VA, US, vol. 12, no. 5, 1 May 2005 (2005-05-01), pages 535-543, XP027798842, ISSN: 1076-6332 [retrieved on 2005-05-01]

## Description

### BACKGROUND

### 1. Technical Field

The present disclosure relates to ultrasound systems and, more specifically, to three-dimensional ultrasound systems configured to modify a display in response to an intraopertive swipe of a user.

### 2. Discussion of Related Art

Ultrasound imaging systems generate a cross-sectional view on an axial plane of an ultrasound transducer array. Depending on the location and orientation of the transducer, the ultrasound image presents differently on a display. It takes thorough knowledge of ultrasound anatomy to map these ultrasound images to real organs.

Surgeons are not trained on mapping ultrasound images to real organs, and thus, a surgeon is not generally capable of mapping ultrasound images to real organs. This prevents surgeons from utilizing ultrasound imaging systems as a tool to guide instruments during a surgical procedure, such as a minimally invasive surgical procedure.

During a minimally invasive surgical procedure, surgery is performed in any hollow viscus of the body through a small incision or through narrow endoscopic tubes (cannulas) inserted through a small entrance wound in the skin or through a naturally occurring orifice. Minimally invasive surgical procedures often require the clinician to act on organs, tissues and vessels far removed from the incision and out of the direct view of the surgeon.

Accordingly, there is a continuing need for instruments and methods to enable a surgeon to visualize a surgical site during a minimally invasive surgical procedure, i.e., intraopertively. Stetten G et al. (Acad. Radiol. 2005; 12:535-543) discloses an ultrasound scanner providing real-time C-mode images of a patient's interior.

### SUMMARY

The invention is defined by the independent claims. Further embodiments of the invention are defined by the dependent claims.

In an aspect of the present disclosure, an ultrasound system includes an ultrasound probe, a processing unit, a camera, and a display. The camera captures an organ or body part's surface view and sends the image stream to the processing unit. The ultrasound probe has a detecting system or sensor configured to detect the position and orientation of the ultrasound probe. The ultrasound probe also has an ultrasound scanner configured to generate a plurality of ultrasound images. The processing unit is in communication with the camera, the detecting system, and the ultrasound scanner. The processing unit is configured to create a three-dimensional data set aligned with the surface view from the camera, based on the position and orientation of the ultrasound probe as it swipes across a surface and when each of the plurality of ultrasound images is generated. The display is in communication with the processing unit and configured to output a view of one subsurface layer from the ultrasound probe, overlaid on the surface view from the camera, as the user swipes the ultrasound probe on a tissue surface. It thus creates a virtual peeling off effect on the display as the user swipes. The display is configured to output a subsurface view of a layer of different depth with another intraoperative swipe on the tissue surface by a user. The user controls the depth of the subsurface view by swiping the ultrasound probe in different directions.

In embodiments, the detecting system may either be a magnetic sensing system or an optical sensing system. A magnetic sensing system includes a positional field generator configured to generate a three-dimensional field. The three-dimensional positional field may be a three-dimensional magnetic field and the sensor may be a magnetic sensor. The positional field generator may be configured to generate the three-dimensional field about a patient or may be configured to generate the three-dimensional positional field from within a body cavity of a patient. The positional field generator may be disposed on a camera or a laparoscope and the sensor may be configured to identify the position and the orientation of the ultrasound probe within the positional field. An optical sensing system includes one or a plurality of markers attached to the end of the ultrasound transducer and the camera. The camera can either be one out of body if the ultrasound probe is used on body surface, or one attached to a laparoscope if the ultrasound probe is used as a laparoscopic tool. The camera communicates the video stream that contains the markers to the processing unit. The processing unit identifies the markers and computes to generate the orientation and position of the ultrasound probe.

In embodiments, the display is configured to overlay a surface image from the camera and a subsurface image layer from the ultrasound system, and align them with right position and orientation.

In aspects of the present disclosure, a method for viewing tissue layers includes capturing surface view from a camera, capturing a plurality of ultrasound images of a patient's body part with an ultrasound probe, recording the position and the orientation of the ultrasound probe with a detecting system, creating a three-dimensional data set having a plurality of subsurface layers aligned to the surface view, intraoperatively swiping the ultrasound probe across patient's body part to view a subsurface layer on a surface view, and other layers of chosen depth with the swipes.

In embodiments, creating the three-dimensional data set includes swiping the ultrasound probe across a patient's body part, associating the plurality of ultrasound images with the position and the orientation of the ultrasound probe, and extracting and viewing the layer of interest from the three-dimensional data set. In some embodiments, swiping the ultrasound probe on the body part replaces a first subsurface layer to display a second subsurface layer that is deeper or shallower than the first layer, depending on the swiping directions.

In particular embodiments, the method includes inserting a surgical instrument into a body cavity of a patient and visualizing the position of the surgical instrument within one of the first and second layers. The method may also include intraoperatively updating the position of the surgical instrument on the display.

In certain embodiments, generating views of subsurface layers includes adjusting the thickness of the view by averaging the ultrasound data over specified depth to enhance visualizing certain features like blood vessels.

The ultrasound system may fill in the gap of ultrasound anatomy between a surgeon and a sonographer enabling a non-invasive method of visualizing a surgical site. In addition, the ultrasound system may provide an intuitive user interface enabling a clinician to use the ultrasound system intraoperatively.

Further, to the extent consistent, any of the aspects described herein may be used in conjunction with any or all of the other aspects described herein.

### BRIEF DESCRIPTION OF THE DRAWINGS

Various aspects of the present disclosure are described hereinbelow with reference to the drawings, wherein:
FIG. 1 is a perspective view of an ultrasound system in accordance with the present disclosure including a camera, an ultrasound probe, a positional and orientation detecting system, a processing unit, and a display;
FIG. 2 is a cut-away of the detail area shown in FIG. 1 illustrating the ultrasound probe shown in FIG. 1 and a laparoscope within a body cavity of a patient;
FIG. 3 is a perspective view of a three-dimensional model generated by the processing unit of FIG. 1 illustrating a plurality of subsurface layers;
FIG. 4 is a view of a first layer on the display of FIG. ; and
FIG. 5 is view of a second layer on the display of FIG. 4 deeper within a body cavity of a patient illustrating the ultrasound transducer swipes on the body part to reveal the second layer with a blood vessel.

### DETAILED DESCRIPTION

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closest to the clinician and the term "distal" refers to the portion of the device or component thereof that is farthest from the clinician.

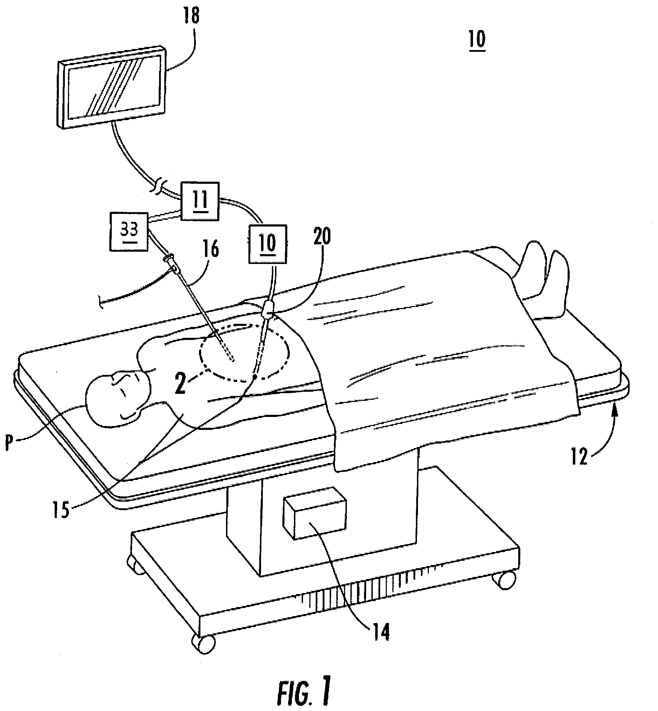

Referring now to FIG. 1, an ultrasound imaging system 10 provided in accordance with the present disclosure includes a camera 33, a position and orientation detecting system or sensor 14, a processing unit 11, a display 18, and an ultrasonic probe 20. The position and orientation detecting system 14 is configured to detect position and orientation with a sensor or marker attached on the ultrasound probe 20 within a region of interest during a surgical procedure.

The ultrasound imaging system is configured to provide cross section views and data sets of a region of interest within a body cavity or on body surface of a patient P on the display 18. A clinician may interact with the ultrasound imaging system 10 and laparoscope 16 attached to a camera 33 to visualize surface and subsurface of a body part within the region of interest during a surgical procedure as detailed below.

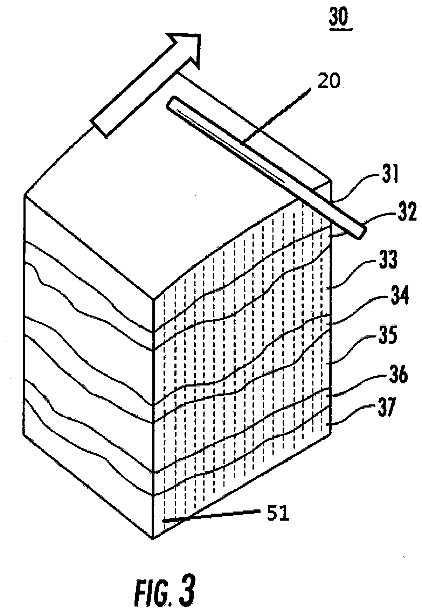

The ultrasound imaging system 10 includes an ultrasound scanner or processing unit 11 that is configured to receive a position and an orientation of the ultrasound probe 20 and a plurality of ultrasound images 51 perpendicular to the surface of the body part from the ultrasound probe 20. The processing unit 11 is configured to relate the position and the orientation of the ultrasound probe 20 with each of the plurality of ultrasound images 51 to generate a 3D ultrasound data set. The processing unit then reorganizes the 3D image pixel data to form a view of one layer in parallel to the scan surface. This layer can be one of the layers 31 to 37 as illustrated in FIG3.

The position and orientation detecting system 14 can either be an optical system that is integrated with the processing unit 11, or a magnetic sensory system that is based on a three-dimensional field. In the optical system case an optical marker 15 is attached on the ultrasound probe, whose position and orientation can be computed with the images captured with camera 33. In the latter case the detecting system 14 has a field generator that generates a three-dimensional field within an operating theater about a patient P. As shown in FIG. 1, the positional field generator is disposed on the surgical table 12 to orientate the patient P within the field. It is within the scope of this disclosure, that the positional field generator is integrated into the surgical table 12. It is also within the scope of this disclosure that the positional field generator may be positioned anywhere within an operating theater or outside the operating theater. The ultrasound imaging system 10 may include a magnetic sensors 15 adhered to the ultrasound probe 20 such that the location and orientation of the ultrasound probe 20 may be used by the processing unit 11 to align the collected three-dimensional data set 30 to the surface view captured by camera 33. An exemplary embodiment of such a magnetic sensing system is disclosed in commonly owned U.S. Patent Application Serial No. 11/242048, filed November 16, 2010, and now published as U.S. Patent No. United States Patent 7835785.

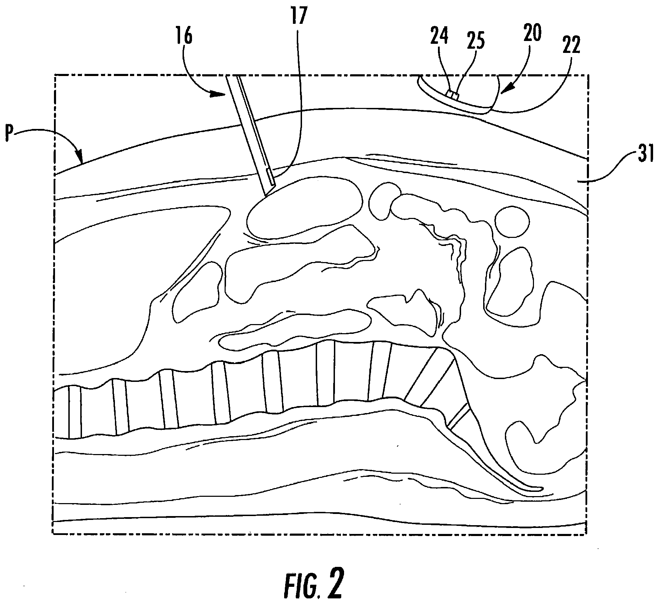

With reference to FIG. 2, the ultrasound probe 20 is adjacent to an outer tissue layer 31 of the patient P. The ultrasound probe 20 includes an ultrasound scanner 22, a position sensor 24, and an orientation sensor 25. The ultrasound scanner 22 is configured to generate and transmit a plurality of ultrasound images of the region of interest to the processing unit 11 (FIG. 1). The processing unit 11 receives the plurality of ultrasound images and receives the position and the orientation of the ultrasound scanner 22 within the field generated by the positional field generator 14 from the position sensor 24 and the orientation sensor 25 at the time each of the plurality of ultrasound images was generated to create a plurality of ultrasound frames. It is also within the scope of this disclosure that the functions of the position sensor 24 and the orientation sensor 25 may be integrated into a single sensor.

It is also within the scope of this disclosure that the plurality of orientation sensors 15, the position sensor 24, and the orientation sensor 25 are image markers whose positioned and orientation may be detected by the positional field generator 14 or a laparoscope. Exemplary embodiments of image markers and positional field generators are disclosed in commonly owned U.S. Patent No. 7,519,218.

In some embodiments, the surgical instrument 16 includes a positional field generator 17 (FIG. 2) that is configured to generate a three-dimensional positional field within a body cavity of the patient P. The sensors 24, 25 identify the position and the orientation of the ultrasound scanner 22 within the three-dimensional positional field generated by the positional field generator 17. It is also contemplated that the position and the orientation of the sensors 24, 25 within the three-dimensional positional fields generated by both positional field generators 14, 17 may be transmitted to the processing unit 11.

With reference to FIG. 3, the processing unit 11 utilizes an image reconstruction algorithm to create a three-dimensional model 30 of the region of interest having a plurality of layers 31-37 that are parallel to the outer surface of the region of interest (i.e., the outer tissue layer 31 of the patient P) from the plurality of ultrasound frames. The processing unit 11 includes an adaptive penetration algorithm that highlights layers within the three-dimensional model 30 that include rich structures (e.g., blood vessels, surfaces of internal organs, etc.). The adaptive penetration algorithm may adjust the thickness of predefined selectable layers 31-37 based on the rich structures within the body cavity (e.g., organs or dense tissue layers). It will be understood that layer 31 may be a surface layer and layers 32-33 may be subsurface layers. An example of this process is when a blood vessel is located in a layer, for example, layer 34 that is not perfectly in parallel to the outer surface 31. In this case the processing unit 11 detects the location of the blood vessel based on the cross section B-mode view 51, and extract layer 34 out of the three-dimensional data set, and reconstruct an image layer that specifically includes the blood vessel to show on display 18. The depth and thickness of the layer 34 is adaptively adjusted based on the detected vessel location.

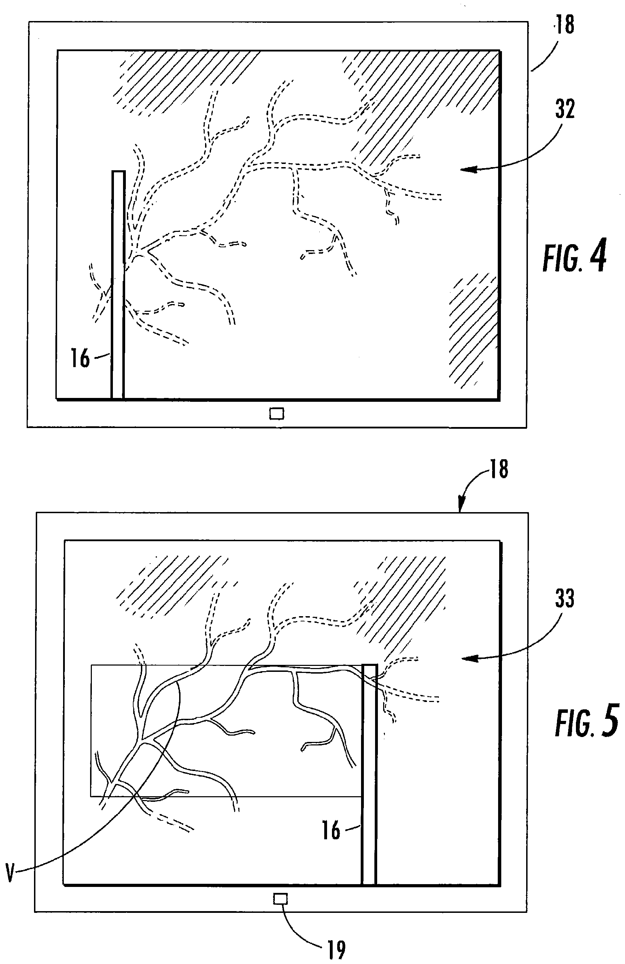

With reference to FIGS. 4 and 5, the display 18 displays a surface view captured by the camera 33, and a subsurface layer view from the processing unit 11 aligned and overlaid to the surface view. The subsurface view is one of the layers 31-37 of the three-dimensional data set 30. The processing unit 11 is configured to detect the movement of the ultrasound probe in a surgical procedure to change the layer 31-37 that is displayed. The display 18 may be configured to enlarge or shrink areas of detail in response to input from a clinician.

With reference to FIGS. 1-5, a method for viewing tissue layers within a patient may be used to position the surgical instrument 16 adjacent a blood vessel V in tissue layer 33. The ultrasonic probe 20 is positioned within the or adjacent to the region of interest to capture a plurality of ultrasonic images of the region of interest within a patient with the ultrasonic scanner 22. The position and orientation of the ultrasonic scanner 22 is recorded with each of the plurality of ultrasound images in the processing unit 11 to create a plurality of image frames. The processing unit 11 creating a plurality of subsurface layers 31-37 parallel to an outer tissue surface of the patient P from the plurality of image frames and outputting a first one of the plurality of layers (e.g., layer 32 as shown in FIG. 4) on the display 18. The clinician may then swipe across the display to view a second, deeper, layer within the region of interest (e.g., layer 33 as shown in FIG. 5). During a surgical procedure, the clinician may insert a surgical instrument into the region of interest while using the ultrasound imaging system 10 to visualize the position of the surgical instrument within the body cavity. As the surgical instrument is repositioned within the region of interest, the position of the surgical instrument is updated on the display 18.

## Claims

1. An ultrasound system (10) comprising:
an ultrasound probe (20) including;
a sensor (15) configured to provide the position and the orientation of the ultrasound probe; and
an ultrasound scanner (22) configured to generate a plurality of ultrasound images (51);
a processing unit (11) in communication with the sensor (15) and the ultrasound scanner (22), the processing unit (11) configured to create a three-dimensional model (30) from the position and the orientation of the ultrasound probe (20) when each of the plurality of ultrasound images (51) is generated; and
a display (18) configured to output a view of a first layer (32) of the three-dimensional model (30);
**characterised in that** the display (18) is further configured to output a view of a second layer (33) of the three-dimensional model (30) different from the first layer (32) in response to an intraoperative swipe across the display (18) by a user.

2. The ultrasound system (10) of claim 1, wherein the first (32) and second (33) layers are parallel to one another.

3. The ultrasound system (10) of claim 1, wherein the second layer (33) is a subsurface layer deeper than the first layer (32).

4. The ultrasound system (10) of any preceding claim further including a positional field generator (14) configured to generate a three-dimensional field about a patient.

5. The ultrasound system (10) of claim 4, wherein the positional field generator (14) generates a three-dimensional magnetic field.

6. The ultrasound system (10) of any preceding claim, wherein the sensor (15) is a magnetic sensor.

7. The ultrasound system (10) of any preceding claim, wherein the sensor is a marker (15) disposed on the ultrasound probe (20).

8. The ultrasound system (10) of claim 7 further including a laparoscope (16) including a positional field generator (17) configured to generate a three-dimensional positional field within a body cavity of a patient, the sensor (15) configured to identify the position and the orientation of the ultrasound probe (20) within the positional field.

9. The ultrasound system (10) of any preceding claim, wherein the display (18) includes a sensor configured to detect an intraoperative swipe across the display.

10. The ultrasound system (10) of any preceding claim, wherein the display (18) is a touch screen display configured to detect an intraoperative swipe across the display.

11. A method for viewing tissue layers (32, 33) comprising:
capturing a plurality of ultrasound images (51) of a body cavity of a patient with an ultrasound probe (20) used on the body surface of the patient; recording the position and the orientation of the ultrasound probe (20) with a sensor (15);
creating a three-dimensional model (30) having a plurality of subsurface layers (32, 33);
viewing a first layer (32) of the plurality of subsurface layers on a display (18); and intraoperatively swiping across the display (18) to view a second layer (33) of the plurality of subsurface layers different from the first layer (32).

12. The method of claim 11, wherein generating the three-dimensional model (30) includes associating the plurality of ultrasound images (51) with the position and the orientation of the ultrasound probe (20).

13. The method of claim 11 or claim 12, wherein swiping the display (18) peels off the first layer (32) of the plurality of subsurface layers (32, 33) to display the second layer (33) that is deeper than the first layer (32).

14. The method of any of claims 11 to 13, wherein creating a plurality of subsurface layers (32, 33) includes adjusting thickness of at least one of the plurality of subsurface layers (32, 33) in response to biological structures (V) within a body cavity of a patient.

## Patentansprüche

1. Ultraschallsystem (10) umfassend:
eine Ultraschallsonde (20) aufweisend:
einen Sensor (15), der konfiguriert ist, um die Position und die Ausrichtung der Ultraschallsonde bereitzustellen; und
einen Ultraschallscanner (22), der konfiguriert ist, um eine Vielzahl von Ultraschallbildern (51) zu generieren;
eine Verarbeitungseinheit (11) in Kommunikation mit dem Sensor (15) und dem Ultraschallscanner (22), wobei die Verarbeitungseinheit (11) konfiguriert ist, um ein dreidimensionales Modell (30) von der Position und der Ausrichtung der Ultraschallsonde (20) zu erschaffen, wenn jedes von der Vielzahl von Ultraschallbildern (51) generiert ist; und
eine Anzeige (18), die konfiguriert ist, um eine Ansicht einer ersten Schicht (32) des dreidimensionalen Modells (30) auszugeben;
**dadurch gekennzeichnet, dass** die Anzeige (18) weiter konfiguriert ist, um eine Ansicht von einer zweiten Schicht (33) des dreidimensionalen Modells (30), die von der ersten Schicht (32) verschieden ist, als Reaktion auf ein intraoperatives Wischen über die Anzeige (18) durch einen Benutzer auszugeben.

2. Ultraschallsystem (10) nach Anspruch 1, wobei die ersten (32) und zweiten (33) Schichten parallel zueinander sind.

3. Ultraschallsystem (10) nach Anspruch 1, wobei die zweite Schicht (33) eine Untergrundschicht tiefer als die erste Schicht (32) ist.

4. Ultraschallsystem (10) nach einem vorstehenden Anspruch, weiter aufweisend einen Positionsfeldgenerator (14), der konfiguriert ist, um ein dreidimensionales Feld um einen Patienten herum zu generieren.

5. Ultraschallsystem (10) nach Anspruch 4, wobei der Positionsfeldgenerator (14) ein dreidimensionales Magnetfeld generiert.

6. Ultraschallsystem (10) nach einem vorstehenden Anspruch, wobei der Sensor (15) ein Magnetsensor ist.

7. Ultraschallsystem (10) nach einem vorstehenden Anspruch, wobei der Sensor eine Markierung (15) ist, die auf der Ultraschallsonde (20) angeordnet ist.

8. Ultraschallsystem (10) nach Anspruch 7, weiter aufweisend ein Laparoskop (16), das einen Positionsfeldgenerator (17) aufweist, der konfiguriert ist, um ein dreidimensionales Positionsfeld innerhalb einer Körperhöhle eines Patienten zu generieren, wobei der Sensor (15) konfiguriert ist, um die Position und die Ausrichtung der Ultraschallsonde (20) innerhalb des Positionsfeldes zu identifizieren.

9. Ultraschallsystem (10) nach einem vorstehenden Anspruch, wobei die Anzeige (18) einen Sensor aufweist, der konfiguriert ist, um ein intraoperatives Wischen über die Anzeige zu detektieren.

10. Ultraschallsystem (10) nach einem vorstehenden Anspruch, wobei die Anzeige (18) eine Touchscreen-Anzeige ist, die konfiguriert ist, um ein intraoperatives Wischen über die Anzeige zu detektieren.

11. Verfahren zum Betrachten von Gewebeschichten (32, 33) umfassend:
Erfassen einer Vielzahl von Ultraschallbildern (51) von einer Körperhöhle eines Patienten mit einer Ultraschallsonde (20), die auf der Körperoberfläche des Patienten verwendet wird;
Aufnehmen der Position und der Ausrichtung des Ultraschallsonde (20) mit einem Sensor (15);
Erschaffen eines dreidimensionalen Models (30) mit einer Vielzahl von Untergrundschichten (32, 33);
Betrachten einer ersten Schicht (32) von der Vielzahl von Untergrundschichten auf einer Anzeige (18); und
intraoperativ Wischen über die Anzeige (18), um eine zweite Schicht (33) von der Vielzahl von Untergrundschichten, die von der ersten Schicht (32) verschieden ist, zu betrachten.

12. Verfahren nach Anspruch 11, wobei Generieren des dreidimensionalen Models (30) aufweist Verknüpfen der Vielzahl von Ultraschallbildern (51) mit der Position und der Ausrichtung der Ultraschallsonde (20).

13. Verfahren nach Anspruch 11 oder Anspruch 12, wobei Wischen der Anzeige (18) die erste Schicht (32) von der Vielzahl von Untergrundschichten (32, 33) ablöst, um die zweite Schicht (33), die tiefer als die erste Schicht (32) ist, anzuzeigen.

14. Verfahren nach einem der Ansprüche 11 bis 13, wobei Erschaffen einer Vielzahl von Untergrundschichten (32, 33) aufweist Einstellen der Dicke von zumindest einer von der Vielzahl von Untergrundschichten (32, 33) als Reaktion auf biologische Strukturen (V) innerhalb einer Körperhöhle eines Patienten.

## Revendications

1. Système à ultrasons (10) comprenant :
une sonde à ultrasons (20) incluant :
un capteur (15) configuré pour fournir la position et l'orientation de la sonde à ultrasons ; et
un scanner à ultrasons (22) configuré pour produire une pluralité d'images par ultrasons (51) ;
une unité de traitement (11) en communication avec le capteur (15) et le scanner à ultrasons (22), l'unité de traitement (11) étant configurée pour créer un modèle tridimensionnel (30) à partir de la position et de l'orientation de la sonde à ultrasons (20) quand chacune de la pluralité d'images par ultrasons (51) est produite ; et
un afficheur (18) configuré pour sortir une vue d'une première couche (32) du modèle tridimensionnel (30) ;
**caractérisé en ce que** l'afficheur (18) est en outre configuré pour sortir une vue d'une seconde couche (33) du modèle tridimensionnel (30) différente de la première couche (32) en réponse à un balayage peropératoire d'u bout à l'autre de l'afficheur (18) par un utilisateur.

2. Système à ultrasons (10) selon la revendication 1, dans lequel les première (32) et seconde (33) couches sont parallèles l'une à l'autre.

3. Système à ultrasons (10) selon la revendication 1, dans lequel la seconde couche (33) est une couche inférieure plus profonde que la première couche (32).

4. Système à ultrasons (10) selon l'une quelconque des revendications précédentes incluant en outre un générateur de champ positionnel (14) configuré pour générer un champ tridimensionnel autour d'un patient.

5. Système à ultrasons (10) selon la revendication 4, dans lequel le générateur de champ positionnel (14) génère un champ magnétique tridimensionnel.

6. Système à ultrasons (10) selon l'une quelconque des revendications précédentes, dans lequel le capteur (15) est un capteur magnétique.

7. Système à ultrasons (10) selon l'une quelconque des revendications précédentes, dans lequel le capteur est un marqueur (15) disposé sur la sonde à ultrasons (20).

8. Système à ultrasons (10) selon la revendication 7, incluant en outre un laparoscope (16) incluant un générateur de champ positionnel (17) configuré pour générer un champ positionnel tridimensionnel à l'intérieur d'une cavité corporelle d'un patient, le capteur (15) étant configuré pour identifier la position et l'orientation de la sonde à ultrasons (20) à l'intérieur du champ positionnel.

9. Système à ultrasons (10) selon l'une quelconque des revendications précédentes, dans lequel l'afficheur (18) inclut un capteur configuré pour détecter un balayage peropératoire d'un bout à l'autre de l'afficheur.

10. Système à ultrasons (10) selon l'une quelconque des revendications précédentes, dans lequel l'afficheur (18) est un afficheur à écran tactile configuré pour détecter un balayage peropératoire d'un bout à l'autre de l'afficheur.

11. Procédé pour visualiser des couches tissulaires (32, 33) comprenant :
la capture d'une pluralité d'images par ultrasons (51) d'une cavité corporelle d'un patient avec une sonde à ultrasons (20) utilisée sur la surface du corps du patient ;
l'enregistrement de la position et de l'orientation de la sonde à ultrasons (20) avec un capteur (15) ;
la création d'un modèle tridimensionnel (30) ayant une pluralité de couches inférieures (32, 33) ;
la visualisation d'une première couche (32) de la pluralité de couches inférieures sur un afficheur (18) ; et
le balayage peropératoire d'un bout à l'autre de l'afficheur (18) pour visualiser une seconde couche (33) de la pluralité de couches inférieures différente de la première couche (32).

12. Procédé selon la revendication 11, dans lequel la génération du modèle tridimensionnel (30) inclut l'association de la pluralité d'images par ultrasons (51) avec la position et l'orientation de la sonde à ultrasons (20).

13. Procédé selon la revendication 11 ou la revendication 12, dans lequel le balayage de l'afficheur (18) ôte la première couche (32) de la pluralité de couches inférieures (32, 33) pour afficher la seconde couche (33) qui est plus profonde que la première couche (32).

14. Procédé selon l'une quelconque des revendications 11 à 13, dans lequel la création d'une pluralité de couches inférieures (32, 33) inclut l'ajustement de l'épaisseur d'au moins une de la pluralité de couches inférieures (32, 33) en réponse à des structures biologiques (V) à l'intérieur d'une cavité corporelle d'un patient.
